Europäisches Patentamt

European Patent Office

Office européen des brevets

(11) Publication number: **0 466 038 A1**

# EUROPEAN PATENT APPLICATION

(21) Application number: **91111148.2**

(22) Date of filing: **04.07.91**

(51) Int. Cl.5: **A61K 31/71, A61K 47/26**

(30) Priority: **12.07.90 US 551354**

(43) Date of publication of application:
**15.01.92 Bulletin 92/03**

(84) Designated Contracting States:
**DE FR GB IT**

(71) Applicant: **E.R. SOUIBB & SONS, INC.**
**P.O.Box 4000**
**Princeton New Jersey 08543-4000(US)**

(72) Inventor: **Tanaka, Makoto**
**2-3-403, 4-chome, Ochiai**
**Tama City, Tokyo 206(JP)**
Inventor: **Takahashi, Masahiro**
**32-7, 1-chome, Kasugacho, Nerima-ku**
**Tokyo 179(JP)**

(74) Representative: **Vossius & Partner**
**Siebertstrasse 4 P.O. Box 86 07 67**
**W-8000 München 86(DE)**

(54) Water-dispersible polyene antifungal complexes.

(57) A water-dispersible antifungal complex and processes for preparation thereof are provided. The complex has: (1) a polyene antifungal agent such as amphotericin B (which is preferred); and (2) one or more water-soluble adjuvants selected from pullulan, polyvinylpyrrolidone (PVP), polyethylene glycol (e.g., PEG 6000), hydroxypropylmethylcellulose (HPMC), hydroxypropylcellulose (HPC), microcrystalline cellulose, gelatin, casein, casein sodium, carboxymethylcellulose sodium, and glycyrrhizin and glycyrrhizin derivatives (which are preferred). The complex is preferred to have a molar ratio from about 1:20 to about 10:1 antifungal agent:adjuvants. The complex may be prepared by the novel process of placing the agent and adjuvant in a basic solution, acidifying to about pH 6.5 to 8.0, and lyophilyzing to form a complex. Alternatively, the complex may be prepared by the novel process of placing the agent and adjuvant in dimethylsulfoxide, adding a nonpolar organic solvent, and filtering to form a complex.

EP 0 466 038 A1

The present invention relates to antifungal formulation complexes that preferably contain amphotericin B as the antifungal agent and one of several water-soluble polymers to promote the release of the antifungal agent into solution upon placement into a solvent, and to processes for making such formulations.

Amphotericin B is a well-known antifungal agent that is supplied commercially as a complex of amphotericin B and desoxycholate colloidally suspended in glucose (Fungizone®). Its use is constrained by a variety of possible toxic side effects, the most serious being nephrotoxicity. These toxic side effects may be related to the inherently poor solubility of this compound in water. Monji et al., "Studies on the absorption, distribution and excretion of radioactivity after intravenous and intraperitoneal administration of $^{14}$C-methyl ester of amphotericin B", The Journal of Antibiotics, Vol. 28, No. 4, 317-325 (1975). Moreover, its poor water-solubility causes poor gastrointestinal absorption. Despite the availability of several new antifungal agents, however, amphotericin B remains the drug of choice for most systemic mycoses in cancer and other immuno-compromised patients.

Many attempts to improve the water-solubility of amphotericin B have been made. Such attempts include complexes with succinic acid (U.S. Patent No. 3,965,090) and with calcium (U.S. Patent No. 3,928,570). In addition, certain derivatives of amphotericin B have been prepared having increased solubility but decreased antifungal potency. Shaffner et al., "Biologically active N-acyl derivatives of polyene macrolide antifungal antibiotics", Antibiot. Chemother., Vol. 11; Kobayashi et al., "In vitro and in vivo comparisons of amphotericin B and N-D-ornithyl amphotericin B methyl ester," Antimicrob. Agents Chemother., Vol. 27. The methyl ester of amphotericin B, described in U.S. Patent No. 3,945,993, exhibits increased water-solibility and decreased toxicity but can be administered intravenously only.

Poorly water-soluble drugs are generally ground to reduce their particle size, since dissolution rate is strongly affected by particle size. However, amphotericin B is hard to pulverize to fine particles with good homogeneity. Moreover, physical and mechanical pulverization of amphotericin B frequently reduces the antifungal potency of its final formulation.

Consequently, researchers have long sought amphotericin B formulations having favorable dispersion while retaining antifungal potency. Improved formulations have long been desired to provide highly water-stable and well-dispersed aqueous solutions with higher bioavailability.

The present invention provides water-dispersible amphotericin B complexes as well as water-dispersible complexes containing other polyene antifungal agents. This invention offers the advantage of rapidly releasing the antifungal agent into water without reducing its activity.

In accordance with the present invention, an antifungal complex is provided, which comprises

(1) a polyene antifungal agent such as amphotericin B (which is preferred), nystatin, trichomycin, pentamycin, candicidin, pimaricin and the like; and

(2) a water-soluble adjuvant selected from glycyrrhizin and its derivatives (which are preferred), pullulan, polyvinylpyrrolidone (PVP), polyethyleneglycol (e.g., PEG 6000), hydroxypropylmethylcellulose (HPMC), hydroxypropylcellulose (HPC), microcrystalline cellulose (e.g., Avicel®), gelatin, casein, casein sodium, and carboxymethylcellulose sodium. A molar ratio from about 1:20 to about 10:1 antifungal agent:adjuvant is preferred (1:9 w/w amphotericin B:dipotassium glycyrrhizinate is most preferred). The term "glycyrrhizin derivatives" refers to glycyrrhizic acid, dipotassium glycyrrhizinate, sodium glycyrrhizinate, diammonium glycyrrhizinate and the like.

Also in accordance with this invention, a novel process is provided wherein a polyene antifungal agent and an adjuvant listed above are placed in a basic solution (a pH of about 11.5 to 12.5 in 1 N sodium hydroxide or 1 N potassium hydroxide is preferred). The pH of this solution is adjusted to about 6.5 to 8.0, preferably by 1.0 N phosphoric acid. The solution is then lyophilized to provide the complex.

Further in accordance with this invention, a novel alternative process of preparation is provided. In this process, a polyene antifungal agent and an adjuvant listed above are heated in dimethylsulfoxide, preferably to about 50 to 60°C. A nonpolar organic solvent (diethyl ether preferred) is added to the solution, and the solvents are removed (filtration preferred) to provide a complex.

Using the above processes, complexes are prepared without reduced antifungal activity that are stable in both solution and solid form at ordinary room temperature and relative humidity.

The antifungal complexes of the present invention are readily dissolved and decomposed at room temperature soon after placement in water to release the antifungal active ingredient. The antifungal active ingredient so dispersed in water has considerably higher antifungal activity than the drug alone. In addition, the adjuvants have negligible toxicity, allowing prescription according to efficacy of the active ingredient rather than toxicity of the adjuvant.

The antifungal complex of the present invention may be formulated in the form of injections, tablets, capsules, suppositories or patches for buccal cavity. Such formulations may further include one or more fillers or diluents, water and/or any other conventional additives. Suppositories and enteric coated capsules and tablets are preferred.

The invention will now be described by the following specific examples. These examples are preferred embodiments of the invention and are meant to be illustrative rather than limiting.

Example 1

In a freeze-drying preparation process for amphotericin B-glycyrrhizin complex (1:4), 135 ml of 1 N sodium hydroxide was added to 600 ml of distilled water. Glycyrrhizic acid (32.8 g) was dissolved very slowly in the solution at 5 to 10°C with agitation. Thereafter, 10 g of amphotericin B with a specific biological activity of 920 mcg/mg was added to the solution incrementally over a period of 10 minutes with vigorous stirring and nitrogen protection. The pH of the solution was adjusted to 6.5 to 8.0 by the addition of about 1 N phosphoric acid in a steady stream. The resultant solution was filtered to obtain a clarified solution. The solution was lyophilized to obtain a solid amphotericin B-glycyrrhizin complex.

These procedures were repeated with molar ratios of 1:0.1, 1:0.5, 1:1, 1:10, and 1:20 amphotericin B:glycyrrhizin.

Example 2

In a solvent method for preparing amphotericin B-glycyrrhizin complex (1:4), 10.0 g of amphotericin B with a specific biological activity of 920 mcg/mg and 35.8 g of dipotassium glycyrrhizinate were dissolved in 100 ml of dimethylsulfoxide at about 50 to 60°C and stirred for 30 minutes. The resultant solution was added to a 4-fold volume of diethyl ether. The precipitate was filtered off, washed with diethyl ether, and dried at 20°C under a residual pressure of 100 mm Hg to give 42.9 g of the amphotericin B-glycyrrhizin complex.

Amphotericin B and glycyrrhizin in other ratios were processed according to the procedures described in Examples 1 and 2 and the dissolution of amphotericin B in physiological saline was determined. The control material used was amphotericin B in bulk form. The dissolution results are as follows:

Table 1:   Dissolution of Amphotericin B into Physiological
           Saline from Amph. B/glycyrrhizin

|          | molar ratio | dissolution (microgram/ml:  %) | | |
|----------|-------------|----------------|-------------|-------------|
|          |             | 5 min.         | 10 min.     | 30 min.     |
| Amph.    | exclusive   | 0.1 (0.2)      | 0.1 (0.3)   | 0.2 (0.5)   |
| Amph.:Gly| 1:0.1       | 0.4 (1.2)      | 0.6 (1.8)   | 0.9 (2.6)   |
|          | 1:0.5       | 0.6 (1.7)      | 0.6 (1.7)   | 0.7 (2.1)   |
|          | 1:1         | 1.7 (5.1)      | 2.5 (7.5)   | 1.5 (4.4)   |
|          | 1:4         | 3.1 (9.2)      | 2.9 (8.6)   | 2.4 (7.1)   |
|          | 1:10        | 6.2 (18.6)     | 5.6 (16.7)  | 6.5 (19.5)  |
|          | 1:20        | 15.6 (47.0)    | 14.5 (43.5) | 12.4 (37.3) |

EP 0 466 038 A1

## EXAMPLE 3

Antifungal formulations containing amphotericin B and pullulan were prepared as described in Examples 1 and 2. The dissolution results are as follows.

Table 2: Dissolution of Amphotericin B from Amph. B/pullulan

| | molar ratio | dissolution (microgram/ml: %) | | |
| --- | --- | --- | --- | --- |
| | | 5 min. | 10 min. | 30 min. |
| Amph. | exclusive | 0.2 (0.7) | 0.3 (0.9) | 0.4 (1.0) |
| Amph.: Pullulan | | | | |
| | 9:1 | 0.4 (1.0) | 0.7 (1.9) | 1.0 (3.0) |
| | 1:1 | 0.6 (1.7) | 0.8 (2.5) | 1.2 (3.5) |
| | 1:4 | 0.6 (1.8) | 1.0 (3.0) | 1.4 (4.0) |
| | 1:9 | 2.2 (6.6) | 3.8 (11.3) | 5.8 (17.1) |

EP 0 466 038 A1

## EXAMPLE 4

Antifungal formulations containing amphotericin B and polyvinylpyrrolidone (PVP) were prepared as described in Examples 1 and 2. The dissolution results are as follows.

Table 3: Dissolution of Amphotericin B from Amph. B/PVP

| | molar ratio | dissolution (microgram/ml: %) | | |
|---|---|---|---|---|
| | | 5 min. | 10 min. | 30 min. |
| Amph. | exclusive | 0.2 (1.0) | 0.3 (0.9) | 0.4 (1.0) |
| Amph.: PVP | | | | |
| | 9:1 | 0.5 (1.5) | 0.8 (2.2) | 1.2 (3.4) |
| | 1:1 | 0.8 (1.8) | 0.9 (2.6) | 1.2 (3.5) |
| | 1:4 | 0.6 (1.7) | 1.0 (3.0) | 1.5 (4.5) |
| | 1:9 | 3.2 (9.6) | 3.0 (8.9) | 3.7 (11.0) |

EP 0 466 038 A1

## EXAMPLE 5

Antifungal formulations containing amphotericin B and PEG 6000 were prepared as described in Examples 1 and 2.  The dissolution results are as follows.

Table 4:  Dissolution of Amphotericin B from Amph. B/PEG 6000

|  | molar ratio | dissolution (microgram/ml: %) | | |
|---|---|---|---|---|
|  |  | 5 min. | 10 min. | 30 min. |
| Amph. | exclusive | 0.2 (1.0) | 0.3 (0.9) | 0.4 (1.0) |
| Amph.:PEG 6000 |  |  |  |  |
|  | 9:1 | 0.5 (1.7) | 1.0 (3.1) | 1.3 (3.9) |
|  | 1:1 | 0.9 (2.6) | 1.2 (3.4) | 1.5 (4.3) |
|  | 1:4 | 1.1 (3.3) | 1.2 (4.0) | 1.4 (4.2) |
|  | 1:9 | 4.0 (1.9) | 4.1 (12.1) | 4.3 (12.8) |

EP 0 466 038 A1

## EXAMPLE 6

Antifungal formulations containing amphotericin B and HPMC were prepared as described in Examples 1 and 2. The dissolution results are as follows.

Table 5: Dissolution of Amphotericin B from Amph. B/HPMC

| molar ratio | dissolution (microgram/ml: %) | | |
|---|---|---|---|
| | 5 min. | 10 min. | 30 min. |
| Amph. exclusive | 0.2 (1.0) | 0.3 (0.9) | 0.4 (1.0) |
| Amph.:HPMC | | | |
| 9:1 | 1.1 (3.3) | 1.4 (4.0) | 1.7 (4.6) |
| 1:1 | 0.4 (1.1) | 0.9 (2.6) | 1.5 (4.3) |
| 1:4 | 0.4 (1.2) | 1.0 (2.8) | 2.2 (6.5) |
| 1:9 | 0.5 (1.4) | 0.9 (2.6) | 2.0 (5.9) |

Example 7

The base Witepsol H-15 was melted at 45°C in a temperature-controlled bath. Equal portions of amphotericin B and dipotassium glycyrrhizinate were added and the mixture was quickly poured into suppository molds and allowed to solidify at room temperature. The target potency was 20 mg amphotericin B/g suppository.

This procedure was repeated with ratios of 1:4 and 1:9 w/w amphotericin B:dispotassium glycyrrhizinate.

These suppositories were tested on white male rabbits weighing from 2.6 to 3.2 kg that received water freely but otherwise fasted for 24 hours prior to administration. Amphotericin B was dosed in 10 mg (activity)/kg and positioned about 2 cm into the rectum. Blood samples of about 1.5 ml were collected from an ear vein of each rabbit at appropriate intervals and evaluated according to procedures in Nilsson-Ehle et al., "Quantitation of amphotericin B with use of High-Pressure Liquid Chromatography," J. Infectious Diseases, Vol. 135, No. 3 (1977). The results are summarized in Table 6.

EP 0 466 038 A1

Table 6:  Serum Concentration of Amphotericin B (ng/ml) in Rabbits
          after Rectal Administration.

The top number in each block in the following table is the mean serum concentration of amphotericin B in ng/ml at the specified time for all of the rabbits studied.  The lower number is the standard error (SE).  The values for the control materials Fungizone® and amphotericin B in bulk form are also provided.

| | Time (hour) | | | | | | | | | |
|---|---|---|---|---|---|---|---|---|---|---|
| Weight Ratio (Amph. B:Gly)[1] | 0.17 | 0.33 | 0.67 | 1.0 | 3.0 | 5.0 | 7.0 | 9.0 | 11.0 | 24.0 |
| 1:1 | – | – | – | 6.5 | 7.4 | 5.5 | 7.3 | 6.9 | 4.8 | 1.9 |
| | – | – | – | 0.2 | 1.6 | 2.3 | 1.2 | 0.6 | 1.4 | 0.8 |
| 1:4 | – | 40.6 | 52.0 | 55.7 | 57.7 | 56.2 | 47.9 | 41.4 | 36.6 | 8.4 |
| | – | 7.2 | 7.1 | 6.4 | 8.9 | 9.6 | 8.7 | 6.8 | 6.5 | 2.9 |
| 1:9 | – | – | – | 123.5 | 114.6 | 105.6 | 95.1 | 81.5 | 77.1 | 12.5 |
| | – | – | – | 14.3 | 8.7 | 3.9 | 3.6 | 3.0 | 3.3 | 2.2 |
| Amph.B (bulk) | – | – | – | 1.0 | 2.3 | 1.5 | 1.0 | 3.3 | 2.7 | 1.3 |
| | – | – | – | 0.4 | 0.3 | 0.1 | 0.2 | 1.4 | 1.1 | 0.1 |
| Fungizone | 7.5 | 17.9 | 24.3 | 25.5 | 24.9 | 27.1 | 23.1 | 18.2 | 15.2 | 3.8 |
| | 2.5 | 2.8 | 6.6 | 6.5 | 8.2 | 8.4 | 10.0 | 8.3 | 7.9 | 2.3 |

[1] "Amph B" refers to amphotericin B; "gly" refers to dipotassium glycyrrhizinate.

(a) a polyene antifungal agent, and
(b) a water-soluble adjuvant selected from pullulan, polyvinylpyrrolidone, polyethylene glycol, hydroxypropylmethylcellulose, hydroxypropylcellulose, microcrystalline cellulose, gelatin, casein, casein sodium, carboxymethylcellulose sodium, glycyrrhizin, and glycyrrhizin derivatives.

2. The complex of Claim 1, wherein the antifungal agent is amphotericin B.

3. The complex of Claim 1, wherein the adjuvant is glycyrrhizin or a glycyrrhizin derivative.

4. The complex of Claim 2, wherein the adjuvant is glycyrrhizin or a glycyrrhizin derivative.

5. The complex of Claim 1, wherein the adjuvant is dipotassium glycyrrhizinate.

6. The complex of Claim 2, wherein the adjuvant is dipotassium glycyrrhizinate.

7. The complex of Claim 6, wherein the formulation comprises 1:9 w/w amphotericin B: dipotassium glycyrrhizinate.

8. The complex of Claim 1, wherein the antifungal agent and the adjuvant are present in a molar ratio from about 1:20 to about 10:1 antifungal agent:adjuvants.

9. A process for preparing a water-dispersible antifungal complex, which comprises:
(a) placing in a basic solution
(i) a polyene antifungal agent, and
(ii) a water-soluble adjuvant selected from pullulan, polyvinylpyrrolidone, polyethylene glycol, hydroxypropylmethyl cellulose, hydroxypropylcellulose, microcrystalline cellulose, gelatin, casein, casein sodium, carboxymethylcellulose sodium, glycyrrhizin and glycyrrhizin derivatives;
(b) adjusting the pH of the solution to about 6.5 to 8.0; and
(c) lyophilizing the solution to form the complex.

10. The process of Claim 9, wherein the antifungal agent is amphotericin B.

11. The process of Claim 9, wherein the adjuvant is glycyrrhizin or a glycyrrhizin derivative.

12. The process of Claim 9, wherein the adjuvant is dipotassium glycyrrhizinate.

13. The process of Claim 9, wherein the basic solution comprises sodium hydroxide.

14. The process of Claim 9, wherein the basic solution has a pH of about 11.5 to 12.5 prior to the adjustment of pH.

15. The process of Claim 9, wherein the adjustment of the pH of the basic solution is carried out by adding phosphoric acid to the basic solution.

16. A process for preparing a water-dispersible antifungal complex, which comprises:
(a) heating a dimethylsulfoxide solution of
(i) a polyene antifungal agent, and
(ii) a water-soluble adjuvant selected from pullulan polyvinylpyrrolidone, polyethylene glycol, hydroxypropylmethylcellulose, hydroxypropyl cellulose, microcrystalline cellulose, gelatin, casein, casein sodium, carboxymethyl cellulose sodium, glycyrrhizin, and glycyrrhizin derivatives;
(b) adding a nonpolar organic solvent to the dimethylsulfoxide solution; and
(c) removing the dimethylsulfoxide and nonpolar solvent to form the complex.

17. The process of Claim 16, wherein the antifungal agent is amphotericin B.

18. The process of Claim 16, wherein the adjuvant is glycyrrhizin or a glycyrrhizin derivative.

19. The process of Claim 16, wherein the adjuvant is dipotassium glycyrrhizinate.

**20.** The process of Claim 16, wherein the nonpolar organic solvent is diethyl ether.

European
Patent Office

EUROPEAN SEARCH
REPORT

Application Number

EP 91 11 1148

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | ANTIBIOTIKI, August 1976, pages 679-685, Moscow, SU; V.A. VEINSTEIN et al.: "Sorption complexes with nystatin and amphotericin B with polyvinylpyrrolidone in non-aqueous solvent systems" * English abstract * | 1-2 | A 61 K 31/71 A 61 K 47/26 |
| X | FR-A-2 342 740 (PHARMACO INC.) * Claims 1,3-6; page 8, lines 11-39; page 11, lines 31-40; page 17, lines 21-23 * | 1-2 | |
| Y | | 3 | |
| Y | DE-A-3 443 242 (YISSUM RESEARCH DEVELOPMENT CO. OF THE HEBREW UNIVERSITY OF JERUSALEM) * Claims 1-3; page 3, lines 16-21; page 9, example 1 * | 3 | |
| A | EP-A-0 147 851 (CHINOIN GYOGYSZER ES VEGYESZETI TERMEKEK GYARA RT) * Claims 1,4,5,9; page 23, example 6 * | 9-10 | |
| A | WO-A-8 505 030 (THE LIPOSOME CO., INC.) * Page 33, lines 1-36 * | 16-17 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 11 September 91 | VENTURA AMAT A. |